# FASCICULE DE BREVET EUROPEEN

(11) **EP 2 509 551 B1**
(45) Date de publication et mention de la délivrance du brevet: **26.11.2014**
(21) Numéro de dépôt: 10801637.9
(22) Date de dépôt: 07.12.2010
(51) Int. Cl.: A61F 9/007, A61F 9/08

(54) **RETINE ARTIFICIELLE COMPRENANT UNE COUCHE EN MATERIAU PHOTOVOLTAIQUE COMPRENANT UN SEMICONDUCTEUR A BASE DE DIOXYDE DE TITANE**
KÜNSTLICHE NETZHAUT MIT FOTOVOLTAIKMATERIALSCHICHT MIT EINEM TITANDIOXID-HALBLEITER
ARTIFICIAL RETINA THAT INCLUDES A PHOTOVOLTAIC MATERIAL LAYER INCLUDING A TITANIUM DIOXIDE SEMICONDUCTOR

(30) Priorité: 08.12.2009 FR 0958744
(43) Date de publication de la demande: 17.10.2012
(73) Titulaire: Centre National de la Recherche Scientifique (CNRS), 75016 Paris (FR); Université de Strasbourg, 67000 Strasbourg (FR)
(72) Inventeur: KELLER, Nicolas, F-67810 Holtzheim (FR); BERNHARDT, Pierre, F-67190 Heiligenberg (FR); ROUX, Michel, F-67000 Strasbourg (FR); ROBE, Anne, F-67400 Illkirch-Graffenstaden (FR); MUTHUKONDA VENKATAKRISHNAN, Shankar, F-67000 Strasbourg (FR); PICAUD, Serge, F-77210 Avon (FR); LEDOUX, Marc., J., F-83136 Néoules (FR); KELLER-SPITZER, Valérie, F-67203 Oberschaeffolsheim (FR); COTTINEAU, Thomas, F-67100 Strasbourg (FR)
(74) Mandataire: Domenego, Bertrand
(86) Numéro de dépôt international: PCT/FR2010/052633
(87) Numéro de publication internationale: WO 2011/070288

(56) Documents cités:
- WO-A2-02/102241
- WO-A2-2004/071338
- US-A1- 2007 005 116
- US-B1- 6 298 270

## Description

La présente invention concerne les rétines artificielles ou prothèses rétiniennes.

La rétine est constituée d'une matrice de photorécepteurs qui captent les signaux lumineux et les transforment en impulsions électriques transmises au réseau de neurones internes de la rétine qui les acheminent ensuite du nerf optique vers les centres visuels du cerveau.

L'objectif d'une rétine artificielle est de restaurer une vision utile chez des patients aveugles atteints d'une dégénérescence des photorécepteurs due par exemple à des rétinopathies pigmentaires ou une dégénérescence maculaire liée à l'âge (plus de 50% des cécités en France). Quand les photorécepteurs dégénèrent, le réseau de neurones internes persiste en partie. La rétine artificielle permet alors de capter les images et de les transformer en signaux électriques capables de stimuler ces neurones, afin de restaurer une vue suffisante pour permettre la locomotion, la reconnaissance des visages, la lecture....

Différentes rétines artificielles ont été développées.

Une des techniques utilise une caméra qui envoie les signaux électriques vers un récepteur, qui assure également l'apport énergétique, installé en périphérie de l'oeil, lui-même relié par un câble à une rétine artificielle sous forme de matrice d'électrodes métalliques (de 16 à 60 électrodes) placée au contact de la rétine, au contact des neurones survivants (Artificial Retina News, automne 2006, hiver 2007, été 2009). Les matériaux utilisés pour ces électrodes sont notamment le platine ou l'oxyde d'iridium. Cette technique se heurte toutefois au problème de miniaturisation des circuits électroniques. Les modèles actuels possèdent 60 électrodes et la limite des 1000 électrodes, considérées comme le minimum pour espérer une reconnaissance des visages, ne devrait pas être atteinte avant plusieurs années.

Une technique alternative consiste à implanter au contact de la rétine des photodiodes qui comprennent des matériaux photosensibles à base de silicium (Besch et al., Br. J. Ophthalmol. 2008, 92(10): 1361-8). Cependant, les photodiodes actuelles requièrent des niveaux lumineux élevés pour émettre un courant suffisant pour provoquer une réponse neuronale. Le rendement des photodiodes actuelles nécessite donc une amplification du courant produit. Cette approche est utilisée par l'entreprise Retinal Implant AG^{®}. Les résultats présentés sont très encourageants. Cependant, il est alimenté par un câble partant de l'élément intraoculaire et, passant en sous-cutané, ressortant à l'arrière de la tête. Ce câble permet l'alimentation en énergie et le contrôle des amplificateurs. Un tel appareillage implique donc, au moins à l'heure actuelle, la persistance d'un câble à travers la paroi oculaire, et une forte limitation voire une absence de mouvements oculaires. L'oeil du sujet est immobilisé. Or, comme une image projetée de façon invariante sur la rétine n'est plus perçue, la rétine et le cerveau humain ne sont pas habitués à travailler à partir d'images fixes, mais au contraire à partir de variations temporelles. La perte de mobilité oculaire ne peut être compensée que partiellement par des mouvements de tête, car ces mouvements ne peuvent pas se faire à la même fréquence que les saccades oculaires. De plus, la présence d'un câble implique également un risque accru d'endophtalmie (infection intraoculaire) ou d'hypotonie (baisse de pression intraoculaire).

Une autre technique est basée sur une prothèse constituée de photodiodes à base de silicium sensibles à l'infrarouge (groupe de Daniel Palanker, Université de Stanford). L'avantage est que pour un niveau d'énergie donné, l'infrarouge est moins nocif que la lumière visible. Cependant, les modèles développés incluent un système d'amplification, tirant son énergie d'une partie de l'illumination infra-rouge. Cette prothèse doit être complétée par des lunettes transformant la lumière visible en lumière infrarouge.

Par ailleurs, il a été proposé d'utiliser des films de nanoparticules de semiconducteurs HgTe (Pappas et al., Nanoletters, 7(2), 513-519, 2007) et PbSe (Zhao et al., Angew. Chem. Int. Ed. Engl. 48, 2407-2410, 2009) pour la stimulation électrique des neurones. Toutefois, ces semiconducteurs sont toxiques pour l'organisme et leur implantation dans le corps pose donc problème.

WO 2004/071338 décrit une rétine artificielle comprenant :
- un substrat (110),
- un film carbone diamant (140) renfermant des dispositifs photovoltaïques (120), chacun de ces dispositifs comprenant une zone active électriquement (122) disposée sur la surface du substrat, en liaison électrique avec une électrode (124).

L'électrode peut comprendre divers matériaux, tel que le titane. De préférence, l'électrode (124) comprend en outre une couche d'oxyde d'iridium revêtant la couche de titane.

L'invention a pour but de proposer une rétine artificielle permettant de remédier aux inconvénients cités, notamment une rétine artificielle ne nécessitant pas la persistance d'un passage de câble à travers la paroi oculaire.

A cet effet, selon un premier aspect, l'invention concerne une rétine artificielle comprenant :
(i) un substrat,
(ii) une première couche disposée sur ledit substrat comprenant des parties en matériau photovoltaïque séparées par au moins une partie en matériau isolant,
(iii) une deuxième couche disposée sur ladite première couche et comprenant des parties en matériau conducteur séparées par au moins une partie en matériau isolant,
dans laquelle le matériau photovoltaïque comprend un semiconducteur à base de dioxyde de titane sous forme de nanotubes.

En effet, l'utilisation d'un semiconducteur à base de dioxyde de titane sous forme de nanotubes permet d'obtenir une rétine artificielle ayant des rendements élevés. La rétine artificielle selon l'invention peut alors émettre un signal suffisant tout en étant biocompatible et donc implantable dans le corps du sujet.

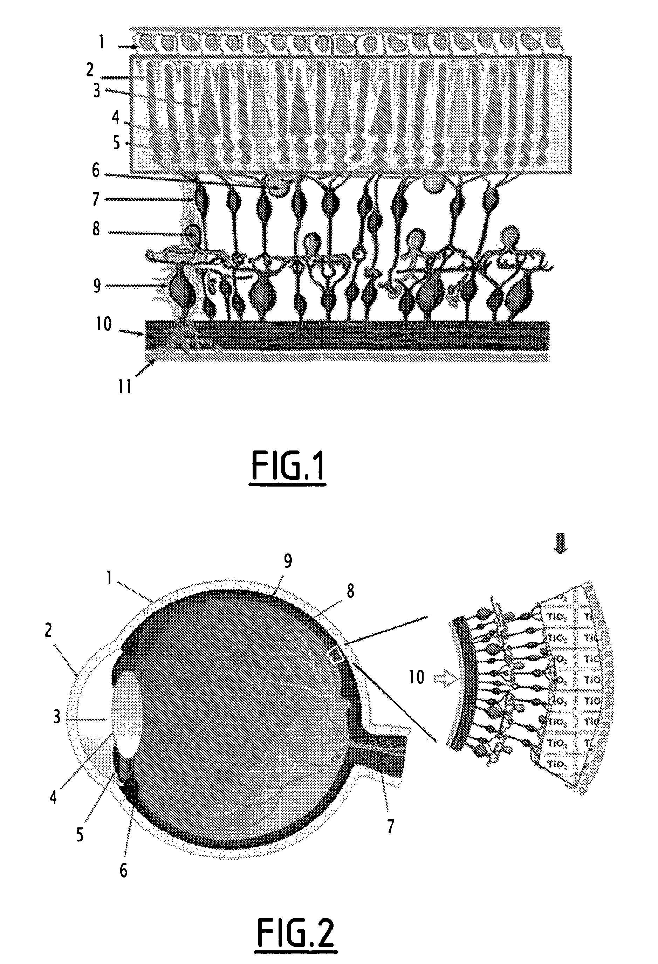

La rétine est une mince structure nerveuse située au fond de l'oeil, couvrant environ 75 % du globe oculaire (figures 1 et 2). Elle assure la partie sensible de la vision en transformant l'image lumineuse focalisée par les lentilles de l'oeil (cornée et cristallin) en un signal électrique, transmis aux centres supérieurs de la vision via le nerf optique, sous forme de potentiels d'action. Une rétine artificielle est un dispositif capable de transformer la lumière en électricité, visant à stimuler certains neurones rétiniens non photosensibles, qui auraient survécu malgré la perte des photorécepteurs naturels.

Une rétine artificielle peut être implantée soit au contact des cellules ganglionnaires, soit à la place des photorécepteurs de la rétine. On parle respectivement de mode épirétinien ou de mode sous-rétinien.

La rétine artificielle selon l'invention sera de préférence utilisée en mode sous-rétinien (figure 2), afin d'utiliser l'ensemble du réseau rétinien persistant après la perte des photorécepteurs.

Avantageusement, la rétine artificielle selon l'invention ne nécessite aucun apport d'énergie externe à l'élément implanté, si ce n'est sous forme de lumière, cette lumière étant typiquement dans les longueurs d'onde du spectre visible et/ou celles du proche infrarouge. Par conséquent, il n'est pas nécessaire de la relier à des dispositifs extérieurs pour son fonctionnement : la rétine artificielle est donc généralement non filaire. Les yeux dans lesquels la rétine est greffée sont ainsi laissés libres de leur mouvement.

De préférence, un système de projection point à point ou de lunettes de type réalité virtuelle sont associés à la rétine artificielle. Le système de projection point à point ou les lunettes assurent l'amplification de l'image en basse lumière et au contraire l'atténuation en forte lumière et facilitent un fonctionnement de la rétine artificielle dans une gamme de luminosité compatible avec une mobilité autonome du sujet chez qui la rétine sera implantée. Aucun câble n'est nécessaire entre la rétine artificielle et le système de projection point à point ou les lunettes.

De préférence, la rétine artificielle a une épaisseur comprise entre 5 et 250 µm, notamment entre 10 et 100 µm, et une longueur et largeur entre 2x2 mm et 10×10 mm, notamment entre 3x3 mm et 5x5 mm. Ces tailles sont en effet adaptées pour implanter la rétine au niveau de la macula (zone centrale de la rétine qui sert pour la vision précise, qui fait environ 2 mm de diamètre).

La rétine artificielle peut être plane, notamment pour des rétines ayant une longueur et largeur de l'ordre de 2x2 mm ou 3x3 mm, avec par exemple une forme circulaire ou elliptique. Elle peut également avoir une forme courbe compatible avec la courbure de l'oeil, par exemple un paraboloïde elliptique. Cette forme courbe est adaptée pour des rétines plus grandes, ayant par exemple une longueur et largeur de l'ordre de 5x5 mm.

Le substrat sert de support à la première couche et à la deuxième couche de la rétine artificielle.

Le substrat peut être opaque. Par exemple, des substrats en polyimide sont adaptés. Un substrat opaque évite le passage de la lumière jusqu'au fond de l'oeil, ce qui limite les réflexions à l'intérieur du globe oculaire, et donc évite une possible dégradation de l'image. Toutefois, le substrat peut également être transparent. Un substrat en diamant peut par exemple être utilisé. Le diamant combine en effet les avantages de forte résistance mécanique, de bonne isolation électrique et de biocompatibilité. Un substrat comprenant plusieurs matériaux peut être utilisé, par exemple un substrat comprenant une couche de diamant conférant les avantages précités et une couche de polyimide pour opacifier le substrat.

La première couche de la rétine artificielle comprend des parties en matériau photovoltaïque séparées par au moins une partie en matériau isolant. Par « parties en matériau photovoltaïque », on entend des parties comprenant ou consistant en un matériau photovoltaïque, et par « partie en matériau isolant », on entend une partie comprenant ou consistant en un matériau isolant.

Le matériau photovoltaïque permet de transformer la lumière incidente en électricité.

Le matériau photovoltaïque de la rétine artificielle selon l'invention comprend un semiconducteur à base de dioxyde de titane sous forme de nanotubes. Généralement, le matériau photovoltaïque de la rétine artificielle comprend au moins 50% de semiconducteur à base de dioxyde de titane sous forme de nanotubes, typiquement au moins 75%, notamment au moins 85%, de préférence au moins 90%.

Un semi-conducteur est un matériau qui a une conductivité électrique intermédiaire entre celle d'un matériau conducteur et celle d'un matériau isolant, à savoir généralement dans la gamme de 10⁻⁵ Ω.m (ohm.mètre) à 10² Ω.m. Le comportement des semi-conducteurs, comme celui des métaux et des isolants est décrit via la théorie des bandes, qui stipule qu'un électron dans un solide ne peut prendre des valeurs d'énergie comprises dans certains intervalles que l'on nomme *bandes permises,* lesquelles sont séparés par d'autres bandes appelées *bandes interdites.* Lorsque la température du solide tend vers le zéro absolu, deux bandes d'énergie permises jouent un rôle particulier: la dernière bande complètement remplie, appelée « bande de valence » et la bande d'énergie permise suivante appelée « bande de conduction ». La bande de valence est riche en électrons mais ne participe pas aux phénomènes de conduction (pour les électrons). La bande de conduction, quant à elle, est soit vide (comme aux températures proches du zéro absolu dans un semi-conducteur) soit semi-remplie (comme dans le cas des métaux) d'électrons. Cependant c'est elle qui permet aux électrons de circuler dans le solide. Dans un semi-conducteur, comme dans un isolant, ces deux bandes sont séparées par une bande interdite, appelée couramment « gap ». L'unique différence entre un semi-conducteur et un isolant est la largeur de cette bande interdite, largeur qui donne à chacun ses propriétés respectives. Dans un isolant la valeur du gap est si grande (aux alentours de 6 eV pour le diamant par exemple) que les électrons ne peuvent passer de la bande valence à la bande de conduction: les électrons ne circulent pas dans le solide. Dans les semi-conducteurs cette valeur est plus petite, généralement de l'ordre de 0,5 à 3,5 eV (1,12 eV pour le silicium, 0,66 eV pour le germanium, 2,26 eV pour le phosphure de gallium, 3,2 eV pour le dioxyde de titane (anatase)). Si on apporte cette énergie (ou plus) aux électrons, par exemple en chauffant le matériau, ou en lui appliquant un champ électromagnétique, ou encore en l'illuminant, les électrons sont alors capables de passer de la bande de valence à la bande de conduction, et de circuler dans le matériau semi-conducteur, tout en laissant un état énergétique non occupé dans la bande de valence, appelé « trou ».

Le semiconducteur est du dioxyde de titane (TiO₂). Avantageusement, le semiconducteur est biocompatible.

Le semiconducteur à base de dioxyde de titane peut être dopé, notamment avec les éléments carbone, azote, soufre et/ou, tungstène, ce qui permet de déplacer le spectre d'absorption du semiconducteur, et donc de la rétine artificielle, vers l'une ou l'autre des parties du spectre, par exemple pour bénéficier d'une meilleure réponse à la lumière visible. Toutefois, l'absorption du semiconducteur dans le visible n'est pas obligatoire. En effet, une absorption dans l'infrarouge est moins nocive pour un niveau énergétique donné. De plus, pour un sujet dont seule une partie des photorécepteurs a été endommagée, l'autre partie des photorécepteurs étant encore fonctionnels, si la rétine artificielle absorbe dans l'infrarouge, il est possible de combiner les effets de la stimulation en infrarouge de la rétine artificielle à ceux de la stimulation à la lumière visible des photorécepteurs encore fonctionnels. Afin de parvenir à une perception optimum, l'image perçue peut être transformée par une caméra externe en une image optimisée pour la résolution de la rétine artificielle implantée et codée dans les longueurs d'onde les mieux absorbées par le semiconducteur à base de dioxyde de titane utilisé.

Le dioxyde de titane est le semiconducteur utilisé en raison de son faible coût, de son rendement photovoltaïque et de sa bonne biocompatibilité.

La rétine artificielle selon l'invention est avantageusement moins onéreuse à préparer que les rétines artificielles comprenant des photodiodes en silicium.

Le dioxyde de titane est avantageusement biocompatible. Le dioxyde de titane est d'ailleurs utilisé comme colorant alimentaire (E171) et le titane est utilisé dans de nombreux types de prothèses (prothèse de hanche, étrier dans les prothèses auditives, reconstruction de la mâchoire, implants crâniens, ancres orthodontiques...). Cette biocompatibilité est un avantage majeur pour son utilisation dans une rétine artificielle qui sera implantée chez un patient.

Le dioxyde de titane existe sous différentes formes cristallines. Le dioxyde de titane peut être sous forme de rutile, d'anatase, de brookite, de srilankite, de TiO₂ α ou de TiO₂ β. Les longueurs d'onde de lumière susceptibles d'activer le dioxyde de titane varient en fonction de sa forme cristalline. Ainsi, le dioxyde de titane sous forme anatase est activé par la fraction UV-A de la lumière solaire, alors que le dioxyde de titane sous forme rutile est activé par la fraction UV-A et par le début du spectre dans le visible de la lumière solaire. Le choix de la forme cristalline du dioxyde de titane est donc lié à la gamme de longueur d'onde pour laquelle la rétine artificielle doit fonctionner.

Le semiconducteur à base de dioxyde de titane dans le matériau est sous forme de nanotubes. Ces nanotubes sont formés par des méthodes connues de l'homme du métier, par exemple par traitement hydrothermal, par exemple entre 130 et 150°C, d'une solution de semiconducteur à base de dioxyde de titane dans une solution aqueuse basique, par exemple d'hydroxyde de sodium. Les procédures décrites dans les publications T. Kasuga, M. Hiramatsu, A. Hoson, T. Sekino, K. Niihara, Langmuir 1998 (14) 3160 - T. Kasuga, M. Hiramatsu, A. Hoson, T. Sekino, K. Niihara, Advanced Materials 1999 (11) 1307, ou D.V. Bavykin, J.M. Friedrich, F.C. Walsh, Advanced Materials 2006 (18) 2807-2824 peuvent par exemple être mises en oeuvre. La structure de nanotubes a une influence sur les propriétés physico-chimiques de la partie en matériau photovoltaïque obtenue, tel que décrit dans l'article T. Tachikawa, S. Tojo, M. Fujitsuka, T. Sekino, T. Majima, Journal of Physical Chemistry B 2006 (110) 14055. Dans les nanotubes, les phénomènes de recombinaison des charges photogénérées sont limités du fait de leur délocalisation le long de la structure unidimensionnelle, qui conduit à améliorer la séparation spatiale des charges de la partie en matériau photovoltaïque. Cette délocalisation accrue entraîne une durée de vie des paires électron-trou plus importante, et donc une densité plus importante de charges photogénérées activées dans le semiconducteur qui pourra être transférée à la deuxième couche, puis aux neurones, pour ainsi obtenir un rendement de la rétine artificielle plus élevé. De plus, en utilisant des nanotubes, la taille des pores (cavité interne du nanotube et espaces entre les nanotubes) est plus grande que celles des pores lorsque des grains sont utilisés (les pores étant alors constituées par les espaces entre les grains), la porosité développée est donc plus accessible, et par conséquent la diffusion du milieu physiologique liquide présent est avantageusement favorisée.

De préférence, les nanotubes utilisés présentent un diamètre extérieur compris entre 50 et 200 nm, typiquement de l'ordre de 100 nm, et un diamètre intérieur de 10 à 100 nm, typiquement de 20 nm à 90 nm. La longueur est fortement dépendante de la durée de synthèse des nanotubes. Des longueurs de nanotubes de 1 à 50 µm, typiquement de 3 à 30 µm sont appropriées pour l'utilisation comme matériau photovoltaïque dans la rétine.

Dans un mode de réalisation, le semiconducteur à base de dioxyde de titane dans le matériau photovoltaïque est sous forme de nanotubes alignés les uns par rapport aux autres. Typiquement, les nanotubes sont alignés les uns par rapport aux autres, les axes des nanotubes étant orthogonaux au plan du substrat Par «nanotubes alignés les uns par rapport aux autres et perpendiculaires au plan du substrat», on entend qu'au moins 80%, voire 95%, des nanotubes sont contenus dans un cône de révolution d'angle de 10°, de préférence 5°, par rapport à la direction orthogonale au substrat.

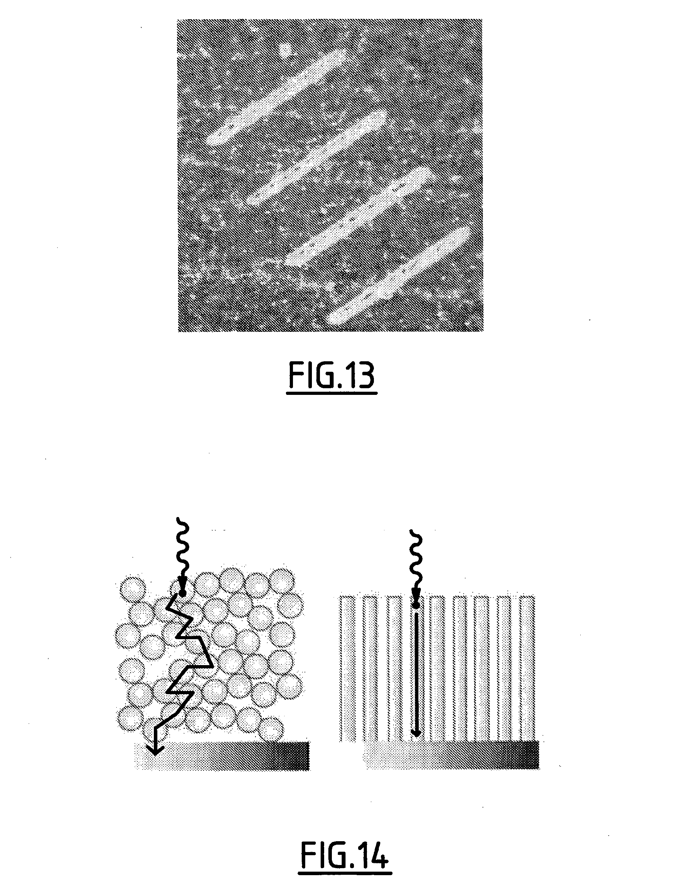

En effet, le flux lumineux traversant des nanotubes alignés est unidirectionnel alors que celui traversant des grains ou des nanotubes enchevêtrés est multidirectionnel. Un flux lumineux unidirectionnel permet avantageusement d'avoir une meilleure absorption de la lumière, car il y a moins de perte par diffusion de la lumière. Le flux lumineux pénètre sur une profondeur (profondeur de pénétration de la lumière) dans les nanotubes et permet la création d'électrons photogénérés. Les électrons photogénérés suivent eux-aussi un parcours rectiligne le long des nanotubes alignés plutôt qu'un parcours erratique d'un grain à l'autre à l'intérieur de la partie en matériau photovoltaïque comprenant un semiconducteur à base de dioxyde de titane sous forme de grains non orientés, comme illustré en figure 14. Cette diminution du parcours des électrons fait que la probabilité de recombinaison des électrons avec les trous est avantageusement diminuée. Enfin les nanotubes présentent une moins grande quantité de joints de grain (qui agissent également comme des centres de recombinaison) que les grains, donc, là encore, la probabilité de recombinaison électron/trou est diminuée, et l'efficacité de la rétine artificielle est donc améliorée. De plus, la résolution maximale de la rétine artificielle est liée à la surface minimale que doit avoir un pixel (cf ci-dessous) pour générer un courant suffisant pour dépolariser le ou les neurones en contact avec la rétine. Or, l'alignement des nanotubes minimise l'espace inter-nanotubes et favorise la pénétration des photons, par rapport à des nanotubes enchevêtrés ou à des grains, ce qui permet d'obtenir des pixels à la fois plus dense et plus épais, et donc de réduire leur surface pour une même efficacité de stimulation, c'est-à-dire d'augmenter la résolution de la rétine artificielle selon l'invention.

Les parties en matériau photovoltaïque comprenant le semiconducteur à base de dioxyde de titane sous forme de nanotubes alignés les uns par rapport aux autres sont généralement obtenues par anodisation électrochimique suivi d'un traitement thermique, généralement sous flux d'air, à une température typiquement comprise entre 400 et 600°C et de préférence pendant 1 à 12 heures, typiquement de 2 à 4 heures.

L'anodisation électrochimique est effectuée en utilisant un système potentiostatique à deux électrodes et en appliquant un potentiel anodique (positif) entre une feuille de titane métallique servant de précurseur du titane et une feuille de platine servant de contre électrode dans un électrolyte approprié. Lors de l'anodisation, le potentiel à appliquer entre les deux électrodes est de préférence compris entre 20 et 100 V, en particulier entre 40 et 60 V, pour synthétiser efficacement les nanotubes. La durée d'anodisation est généralement comprise entre 10 minutes et 100 heures, typiquement entre 15 minutes et 2 heures.

Lorsque le substrat est du titane, l'anodisation électrochimique peut être effectuée directement sur le substrat. Par contre, lorsque le substrat n'est pas en titane (par exemple, substrats en silicium, verre, matériau transparent polymères, etc...), une étape de dépôt de titane sur le substrat est généralement ajoutée préalablement à l'anodisation électrochimique, le titane métallique présent à la surface du substrat obtenu permettant l'étape ultérieure d'anodisation électrochimique.

Ce dépôt de titane peut avantageusement être réalisée par pulvérisation par magnétron (« magnétron sputtering » en anglais), qui consiste à bombarder par les ions issus d'un plasma d'argon, une cible de titane métallique, dont les atomes de titane vont être arrachés par les ions et vont se déposer sur le substrat placé en vis-à-vis de la cible.

Différentes épaisseurs de titane déposé sur le substrat peuvent ainsi être obtenues, typiquement entre 100 nm et 3000 nm d'épaisseur. Des épaisseurs supérieures à 500 nm sont préférées pour permettre une bonne formation des nanotubes par anodisation électrochimique. Plusieurs paramètres de la pulvérisation par magnétron permettent de varier faire l'épaisseur du dépôt : le temps de pulvérisation, l'intensité du courant de plasma, la température du substrat et la distance cible-substrat qui dépend de chaque appareil.

Les parties en matériau photovoltaïque comprenant le semiconducteur à base de dioxyde de titane sous forme de nanotubes alignés les uns par rapport aux autres peuvent être caractérisées par les techniques de microscopie habituelles, telles que les microscopies électroniques à balayage et à transmission, par la diffraction des rayons X, ainsi que par la spectroscopie UV-visible.

Dans un mode de réalisation particulier, les nanotubes alignés les uns par rapport aux autres sont modifiés avec un sensibilisateur (« sensitizer » en anglais), c'est à dire une molécule ou un matériau permettant d'absorber des photons d'énergie plus faible que la bande interdite du TiO₂. Cette modification peut par exemple être effectuée par greffage chimique dudit sensibiliseur sur les nanotubes, par des méthodes connues de l'homme du métier. La présence du sensibilisateur permet d'augmenter l'absorption de lumière dans le visible (longueur d'onde > 400 nm) par les parties en matériau photovoltaïque, et donc par la rétine. Le sensibilisateur peut notamment être:
- un semi-conducteur ayant une bande interdite inférieure à celle du TiO₂, tel que par exemple WO₃, CdS et CdSe,
- un colorant organométallique, tel que le polypyridine de ruthénium
- un colorant organique, tels que les bore-dipyrrométhène, aussi appelés Bodipy.

En plus ou à la place de la modification avec un sensibilisateur susmentionnée, les nanotubes alignés les uns par rapport aux autres peuvent être dopés avec de l'azote, ce qui permet d'augmenter l'absorption de lumière dans le visible (longueur d'onde > 400 nm) par les parties en matériau photovoltaïque, et donc par la rétine. La préparation de nanotubes alignés les uns par rapport aux autres dopés avec de l'azote peut également être effectuée par anodisation électrochimique, notamment en ajoutant de l'urée N₂COH₄ dans l'électrolyte, typiquement entre 0,1 et 0,5 mol.L⁻¹. Les teneurs les plus élevées donnent les taux de dopage les plus importants. Le traitement thermique qui suit l'anodisation peut être effectué dans les mêmes conditions que celles susmentionnées, excepté que le flux d'air est de préférence remplacé par un flux d'azote.

Généralement, les largeurs et longueurs des parties en matériau photovoltaïque sont indépendamment les unes des autres comprises entre 10 µm et 300 µm, notamment entre 20 µm et 200 µm. Pour une rétine de surface donnée, plus les longueurs et/ou largeurs des parties en matériau photovoltaïque sont élevées, moins la rétine artificielle comporte de parties en matériau photovoltaïque (et donc de pixels comme explicité plus loin), et plus la reconstitution d'image grâce à la rétine artificielle est grossière. C'est pourquoi les largeurs et longueurs des parties en matériau conducteur sont généralement inférieures ou égales à 200 µm.

La première couche de la rétine artificielle comprend des parties en matériau photovoltaïque séparées par au moins une partie en matériau isolant.

Le matériau isolant de la première couche est un matériau isolant compatible avec le substrat, les parties en matériau photovoltaïque et la deuxième couche, notamment en permettant de minimiser les tensions d'interface. Un matériau isolant a une résistivité est typiquement supérieure à 1 Ω.m, typiquement 10² Ω.m (ohm.mètre), notamment 10⁶ Ω.m, de préférence 10¹⁰ Ω.m, à 300 K.

Le matériau isolant peut notamment être du diamant isolant, une céramique ou un polymère isolant, par exemple un polyimide ou une résine époxyde, silicone ou polyéthylène expansé.

La première couche a généralement une épaisseur comprise entre 500 nm et 10 µm. Typiquement, les parties en matériau isolant et les parties en matériau photovoltaïque de la première couche ont la même épaisseur, qui est celle de la première couche.

Dans les parties en matériau photovoltaïque, lorsque le semiconducteur à base de dioxyde de titane est activé par de la lumière, des charges positives sont générées. Ces charges photogénérées positives, généralement appelées 'trous', se situent énergétiquement dans la bande de valence du matériau semiconducteur. Plus précisément, un semiconducteur possède une structure électronique à bande interdite. Lorsqu'il est soumis à un rayonnement de photons d'énergie au moins égale à celle de la bande interdite, un électron peut passer de la bande de valence à celle de conduction. Il y a alors création d'un trou au niveau de la bande de valence. Ces charges positives possèdent un fort pouvoir oxydant et sont susceptibles de dégrader les neurones au contact.

Par conséquent, la rétine artificielle selon l'invention comporte également une deuxième couche comprenant des parties en matériau conducteur séparées par au moins une partie en matériau isolant. Par «parties en matériau conducteur », on entend une partie comprenant ou consistant en un matériau conducteur. Un matériau conducteur est un matériau de faible résistance, dont la résistivité est typiquement inférieure à 10⁻⁵ Ω.m (ohm.mètre), notamment 10⁻⁶ Ω.m, de préférence 10⁻⁷ Ω.m, à 300 K, qui permet de véhiculer le courant d'un point à un autre.

Au niveau de l'interface entre les parties en matériau conducteur de la deuxième couche et les parties en matériau photovoltaïque de la première couche, l'interface semiconducteur à base de dioxyde de titane/conducteur joue le rôle d'une barrière de Schottky et empêche le transfert des charges positives aux neurones en contact avec la rétine artificielle. Une barrière de Schottky est la barrière de potentiel (c'est à dire la différence de potentiel) formée à l'interface entre un conducteur et un semiconducteur. La hauteur de cette barrière reflète l'écart entre la position énergétique de la limite de la bande de conduction et le niveau de Fermi du conducteur. La deuxième couche de la rétine artificielle permet ainsi d'éviter la dégradation des neurones par les charges positives.

La deuxième couche a généralement une épaisseur de 0,5 à 20 nm, notamment de 1 à 10 nm, de préférence de 2 à 5 nm.

Le matériau conducteur est un matériau conducteur compatible avec la première couche, qui permet notamment de minimiser les tensions d'interface entre la première et la deuxième couche.

Le matériau conducteur peut être un métal, par exemple de l'or ou du platine. Le matériau conducteur peut également être du nitrure de titane ou de l'oxyde d'iridium. De préférence, le matériau conducteur est de l'or. Le matériau conducteur peut également être en diamant conducteur (diamant dopé), qui présente avantageusement une meilleure résistance mécanique que l'or et une excellente biocompatibilité. Le matériau conducteur peut également être en polymère conducteur, par exemple en parylène-C(poly-dichloro-diparaxylylène).

Dans un mode de réalisation, les parties en matériau conducteur ont une épaisseur égale à celle des parties en matériau isolant. Dans un autre mode de réalisation, les parties en matériau isolant ont une épaisseur supérieure à celle des parties en matériau conducteur et les parties en matériau isolant font saillie sur la deuxième couche. Dans un autre mode de réalisation, les parties en matériau conducteur ont une épaisseur supérieure à celle des parties en matériau isolant et les parties en matériau conducteur font saillie sur la deuxième couche, notamment de 3 à 50 µm, par exemple de 10 à 50 µm. L'épaisseur des parties en matériau conducteur est alors supérieure, par exemple de 10 à 50 µm, à celle(s) de la(des) partie(s) isolante(s) de la deuxième couche. La rétine artificielle de ce mode de réalisation est plus efficace grâce à l'accroissement de la surface de contact entre les parties en matériau conducteur et les neurones, et donc de la quantité de charge pouvant être transmise.

Généralement, les largeurs et longueurs des parties en matériau conducteur sont indépendamment les unes des autres comprises entre 10 µm et 50 µm, notamment entre 20 µm et 30 µm. Pour des longueurs ou largeurs inférieures à 10 µm, la résolution est généralement insuffisante et la densité de courant est généralement trop forte, ce qui augmente les risques de dommages pour les neurones au contact de la rétine. Pour une rétine artificielle de surface donnée, plus les longueurs et/ou largeurs des parties en matériau conducteur sont élevées, moins la rétine artificielle comporte de parties en matériau conducteur (et donc de pixels comme explicité ci-dessous) et plus la reconstitution d'image grâce à la rétine artificielle est difficile. C'est pourquoi les largeurs et longueurs des parties en matériau conducteur sont généralement inférieures à 50 µm. Généralement, les surfaces des parties en matériau conducteur sont inférieures à celles des parties en matériau photovoltaïque.

Le matériau isolant de la deuxième couche est un matériau isolant compatible avec les parties en matériau conducteur et la première couche (parties en matériau photovoltaïque et partie isolante de la première couche), notamment en permettant de minimiser les tensions d'interface. Ce matériau isolant peut notamment être du diamant isolant ou un polymère isolant. Généralement, le matériau isolant des première et deuxième couches est le même, ce qui simplifie la préparation de la rétine artificielle.

Dans un mode de réalisation, le matériau conducteur est un métal, notamment de l'or, et le matériau isolant de la première et de la deuxième couche est un polymère isolant. Dans un autre mode de réalisation, le matériau conducteur est un diamant conducteur et le matériau isolant de la première et de la deuxième couche est un diamant isolant.

Lorsque la deuxième couche comporte plus d'une partie en matériau isolant, les longueurs et largeurs des parties en matériau isolant de la deuxième couche sont indépendamment les unes des autres généralement comprises entre 1 µm et 50 µm, notamment entre 5 µm et 20 µm.

Les parties en matériau isolant des première et deuxième couches permettent la pixellisation de la rétine artificielle. Pour que la rétine artificielle permette au sujet chez qui elle est implantée de distinguer des formes, de reconnaître un visage, de lire une lettre, il est en effet nécessaire que la rétine soit structurée pour qu'elle comprenne plusieurs pixels.

Cette pixellisation est obtenue grâce à la structure des première et deuxième couches. Plus précisément, chaque sous-ensemble [substrat / partie en matériau photovoltaïque / partie en matériau conducteur], constitue un pixel. La rétine artificielle comporte autant de pixels que de sous-ensembles [substrat / partie en matériau photovoltaïque / partie en matériau conducteur]. Chaque pixel doit être isolé électriquement des autres pixels, cette isolation étant assurée par les parties en matériau isolant. Ainsi, lorsque seules certaines zones de la rétine artificielle reçoivent de la lumière, seuls certains des sous-ensembles précités (certains pixels) transforment la lumière en un signal électrique transmis de façon localisée aux neurones rétiniens en contact avec ces sous-ensembles, ce qui permet de reconstituer une image.

Plus la rétine artificielle comporte de pixels, plus les formes peuvent être distinguées facilement par le sujet chez qui la rétine a été implantée. Typiquement, la rétine artificielle comporte entre 600 et 10 000 parties en matériau conducteur, notamment de 1000 à 6000.

La première couche est une couche discontinue comportant dans le plan au moins une partie en matériau isolant et des parties en matériau photovoltaïque. La deuxième couche est une couche discontinue comportant dans le plan au moins une partie en matériau isolant et des parties en matériau conducteur. Généralement, ces couches ne comportent qu'une partie en matériau isolant continue dans laquelle sont dispersées les parties en matériau photovoltaïque (pour la première couche) ou conducteur (pour la deuxième couche). Dans un autre mode de réalisation, la première et/ou deuxième couche comporte(nt) plusieurs parties en matériau isolant séparées les unes des autres par les parties en matériau photovoltaïque (pour la première couche) ou conducteur (pour la deuxième couche).

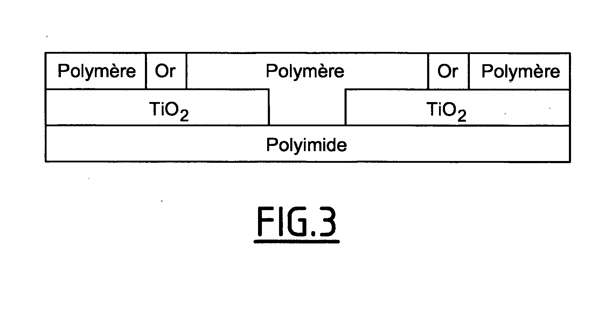

Les parties en matériau photovoltaïque et les parties en matériau conducteur de la rétine artificielle sont typiquement superposées les unes aux autres, c'est-à-dire qu'en observant une coupe verticale de la rétine artificielle, une partie en matériau conducteur est disposée au dessus d'une partie en matériau photovoltaïque (et non pas au dessus d'une partie en matériau isolant de la première couche), comme illustré sur la figure 3. Chaque partie en matériau photovoltaïque associée à une partie en matériau conducteur forme avec le substrat un pixel. Typiquement, la rétine comprend autant de parties en matériau conducteur que de parties en matériau photovoltaïque. Une rétine artificielle comportant un nombre de pixels donné comporte donc ce même nombre de parties en matériau photovoltaïque et ce même nombre de parties en matériau conducteur.

Généralement, la partie en matériau conducteur est de surface inférieure à celle de la partie en matériau photovoltaïque sous-jacente, afin de concentrer les charges émises vers un seul neurone ou un petit groupe de neurones.

La figure 3 illustre ce mode de réalisation, pour le cas d'une rétine artificielle comprenant un substrat (non représenté), une première couche comprenant au moins une partie en matériau isolant en polymère isolant et des parties en matériau photovoltaïque en dioxyde de titane (formées par dépôt couche par couche de dioxyde de titane et de polyimide) et une deuxième couche comprenant des parties en matériau conducteur en or séparées par au moins une partie en matériau isolant en polymère isolant. Des pixels formés d'une surface localisée de TiO₂, par exemple des zones de 200 µm x 200 µm, et d'une surface localisée d'or, par exemple de 10 µm x 10 µm sont représentés. Chaque pixel est isolé des autres pixels grâce à un polymère isolant. Deux pixels sont ainsi représentés sur la figure 3.

Dans un mode de réalisation, la rétine artificielle comporte en outre une troisième couche disposée sur la deuxième couche et comprenant un matériau favorisant l'adhésion avec les cellules. Par exemple, la rétine peut comporter une couche de polylysine, d'éléments de la matrice extracellulaire (laminines, collagènes, fibronectine, vitronectine, ...), ou de cell-tak™ (protéines polyphénoliques produites par la moule *Mytilus edulis,* distribué par BD Biosciences). Toutefois, cette troisième couche n'est pas obligatoire car la rétine établit normalement un contact stable avec les tissus à son contact.

Dans un mode de réalisation, la rétine artificielle comprend en outre une couche constituée d'un matériau conducteur disposée entre le substrat et la première couche.

Cette couche est de préférence en matériau transparent conducteur (TCO « Transparent Conductive Oxide » en anglais), par exemple en en oxyde d'étain dopé au fluor (FTO) ou en oxyde d'étain dopé à l'Indium (ITO).

Selon un deuxième aspect, l'invention concerne un procédé de préparation de la rétine artificielle précitée comprenant les étapes consistant à :
a) déposer un semiconducteur à base de dioxyde de titane sur un substrat pour former une couche en matériau photovoltaïque,
b) déposer une couche en matériau conducteur sur la couche en matériau photovoltaïque obtenue à l'étape a) pour former une couche en matériau conducteur,
c) insérer au moins une partie en matériau isolant dans la couche en matériau conducteur et dans la couche en matériau photovoltaïque pour former la première et la deuxième couche.

Dans l'étape a), le dépôt du semiconducteur à base de dioxyde de titane est typiquement un dépôt couche par couche.

La technique de dépôt couche par couche permet d'obtenir une couche en matériau photovoltaïque à partir de laquelle seront formées les parties en matériau photovoltaïque de la première couche de la rétine artificielle. La couche en matériau photovoltaïque est la couche à partir de laquelle est formée la première couche comprenant des parties en matériau photovoltaïque séparées par au moins une partie en matériau isolant. La couche en matériau photovoltaïque comprend donc les matériaux photovoltaïques précités.

Le dépôt couche par couche est une technique de préparation de couches minces, formées par déposition de façon alternative de couches de matériaux chargés de façon opposée (assemblage par interactions électrostatiques), avec des étapes de rinçage entre chaque dépôt d'une couche.

Une couche est réalisée à partir d'une solution comprenant le semiconducteur à base de dioxyde de titane ou un précurseur dudit semiconducteur. Un précurseur est un composé chimique qui, suite au dépôt couche par couche et/ou après réaction chimique, est transformé est semiconducteur à base de dioxyde de titane. Par exemple, des alkoxydes de titane, tel que le tétra-isopropoxyde de titatane (TTIP) peuvent être utilisés lors du dépôt couche par couche. La solution est généralement aqueuse. Le pH de la solution est adapté en fonction du point isoélectrique du semiconducteur à base de dioxyde de titane pour ajuster la charge du semiconducteur. Le point isoélectrique est le pH auquel le semiconducteur à base de dioxyde de titane a une charge globale neutre. Si la solution a un pH inférieur au point isoélectrique du semiconducteur à base de dioxyde de titane, sa charge globale est positive et il forme donc une couche de charge positive sur le substrat. Inversement, si la solution a un pH supérieur au point isoélectrique du semiconducteur à base de dioxyde de titane, sa charge globale est négative et il forme donc une couche de charge négative sur le substrat. Chaque semiconducteur à base de dioxyde de titane a un point isoélectrique qui lui est propre. Chaque forme cristalline de dioxyde de titane a son propre point isoélectrique.

Ensuite, une couche de charge opposée est formée à partir d'une solution comprenant un composé de charge opposée à celle du semiconducteur à base de dioxyde de titane (ou de son précurseur).

Selon les conditions expérimentales et la nature du semiconducteur à base de dioxyde de titane, la couche comprenant le semiconducteur à base de dioxyde de titane peut constituer la couche négative ou la couche positive. Dans un mode de réalisation, les couches de charge positive et négative sont toutes les deux formées à partir de semiconducteur à base de dioxyde de titane (de charge globale opposées).

Dans le mode de réalisation dans lequel le semiconducteur à base de dioxyde de titane ne forme qu'une seule couche (soit la couche de charge positive, soit la couche de charge négative), le(s) composé(s) qui forme(nt) les couches intermédiaires entre les couches de semiconducteur à base de dioxyde de titane est(sont) biocompatible(s).

Les couches positives sont réalisées à partir d'une solution comprenant un sel, par exemple une solution d'halogénure de diallyldiméthylammonium (dépôt du cation diallyldiméthylammonium), ou un composé protoné dans les conditions de pH mise en oeuvre lors du dépôt, par exemple un halogénure de polyéthylèneiminium (dépôt du cation polyéthylèneiminium). Les chlorure, bromure et iodure sont les halogénures préférés. D'autres sels sont utilisables pour former les couches positives (ou négatives lorsque le semiconducteur à base de dioxyde de titane forme la couche de charge positive), par exemple ceux mentionnés dans G. Decher, J.B. Schlenoff, « Multilayer thin films sequential assembly of nanocomposite materials », Wiley-VCH Eds, Weinheim, Germany, 2003, 524 pages.

Les différentes couches de même charge dans la couche en matériau photovoltaïque peuvent chacune comprendre des composés de nature différente. Par exemple, il est possible de réaliser une première couche à charge positive de polyéthylèneiminium, puis une couche à charge négative de dioxyde de titane, puis une couche à charge positive de diallyldiméthylammonium, puis une couche négative de dioxyde de titane.

Dans un mode de réalisation, l'étape a) comprend les étapes suivantes :
i) immerger un substrat dans une solution aqueuse d'halogénure de polyéthylèneiminium ou d'halogénure de diallyldiméthylammonium,
ii) rincer le substrat obtenu à l'étape i) à l'eau,
iii) immerger le substrat obtenu à l'étape ii) dans une solution aqueuse d'halogénure de polyéthylèneiminium ou d'halogénure de diallyldiméthylammonium,
iv) rincer le substrat obtenu à l'étape iii) à l'eau,
v) immerger le substrat obtenu à l'étape iv) dans une solution comprenant du semiconducteur à base de dioxyde de titane,
vi) rincer le substrat obtenu à l'étape v) à l'eau,
dans lequel l'enchaînement des étapes iii), iv), v) et vi) peut être répété, ce par quoi on dépose couche par couche le semiconducteur à base de dioxyde de titane.

La technique de dépôt couche par couche est une technique simple et peu coûteuse permettant de contrôler l'épaisseur de chaque couche. Généralement, la couche en matériau photovoltaïque est préparée par dépôt de 2 à 10, notamment 2 à 5 couches de semiconducteur à base de dioxyde de titane sur le substrat.

Pour réaliser l'étape a), d'autres méthodes que le dépôt couche par couche peuvent être envisagées pour préparer la couche en matériau photovoltaïque, par exemple par dépôt de couche mince de semiconducteur à base de dioxyde de titane, notamment par évaporation ou pulvérisation cathodique (sputtering en anglais), ou par lithographie.

L'étape b) est réalisée de préférence par dépôt physique ou chimique en phase vapeur ou par lithographie. Le dépôt physique en phase vapeur est une technique qui consiste à déposer sur un substrat une couche mince sous vide par condensation du matériau constitutif de la couche en matériau conducteur sous forme vaporisé. Le dépôt chimique en phase vapeur est un technique qui consiste à exposer un substrat à un ou plusieurs précurseurs en phase gazeuse, qui réagissent et/ou se décomposent à la surface du substrat pour générer le dépôt désiré. L'étape b) peut également être réalisée par imprégnation d'un sel précurseur du matériau conducteur, puis transformation de ce sel précurseur en matériau conducteur.

La couche en matériau conducteur est la couche à partir de laquelle est formée la deuxième couche comprenant des parties en matériau conducteur séparées par au moins une partie en matériau isolant. La couche en matériau conducteur comprend donc les matériaux conducteurs précités.

Le procédé de préparation de la rétine artificielle comprend en outre une étape c) consistant à insérer au moins une partie en matériau isolant dans la couche en matériau photovoltaïque (obtenue à l'étape a)) et dans la couche en matériau conducteur (obtenue à l'étape b)).

Typiquement, l'étape c) comprend une étape de microgravure, qui permet d'enlever des zones localisées de matériau photovoltaïque et de matériau conducteur par un procédé physique (par exemple avec un laser ou par application de lumière UV) ou chimique de manière à obtenir en inverse le marquage désiré. Ensuite, au moins un matériau isolant est inséré dans ces zones pour former les parties en matériau isolant des première et deuxième couches.

Le matériau isolant peut être inséré par lithographie.

Généralement, pour simplifier le procédé, le même matériau isolant est inséré dans la couche en matériau photovoltaïque et dans la couche en matériau conducteur, et la rétine artificielle obtenue comporte donc des parties en matériau isolant de même nature dans les première et deuxième couches.

### FIGURES

La figure 1 représente une coupe de la rétine. 1 : Pigment épithélium - 2 : Bâtonnets - 3 : Cônes - 4 : Membrane limitante externe - 5 : Cellules de Müller - 6 : Cellules horizontales - 7 : Cellules bipolaires - 8 : Cellules amacrine - 9 : Cellules ganglionnaires - 10 : Couche de fibres nerveuses - 11 : - Membrane limitante interne.
La figure 2 représente une coupe d'un oeil et montre la localisation de la rétine artificielle en mode sous rétinien. 1 : Sclère - 2 : Cornée - 3 : Pupille - 4 : Lentille - 5 : Iris - 6 : Corps ciliaire - 7 : Nerf optique - 8 : - Rétine - 9 : Choroïde - 10 : Lumière.
La figure 3 représente un schéma d'une coupe verticale d'une rétine artificielle illustrant une technique de pixellisation obtenue par microgravure pour le cas d'une rétine artificielle comprenant, sur un substrat (non représenté), une première couche comprenant une partie en matériau isolant en polymère isolant et des parties en matériau photovoltaïque en dioxyde de titane (formée par dépôt couche par couche de dioxyde de titane et de polyimide) et une deuxième couche comprenant une partie en matériau isolant en polymère isolant et des parties en matériau conducteur en or. Deux pixels comprenant chacun une partie en matériau photovoltaïque de TiO₂, par exemple sur une zone de 200 µm x 200 µm, et une partie en matériau conducteur en or, par exemple de surface donnée de 10 µm x 10 µm sont représentés, chaque pixel étant isolé des autres pixels grâce à un polymère isolant.

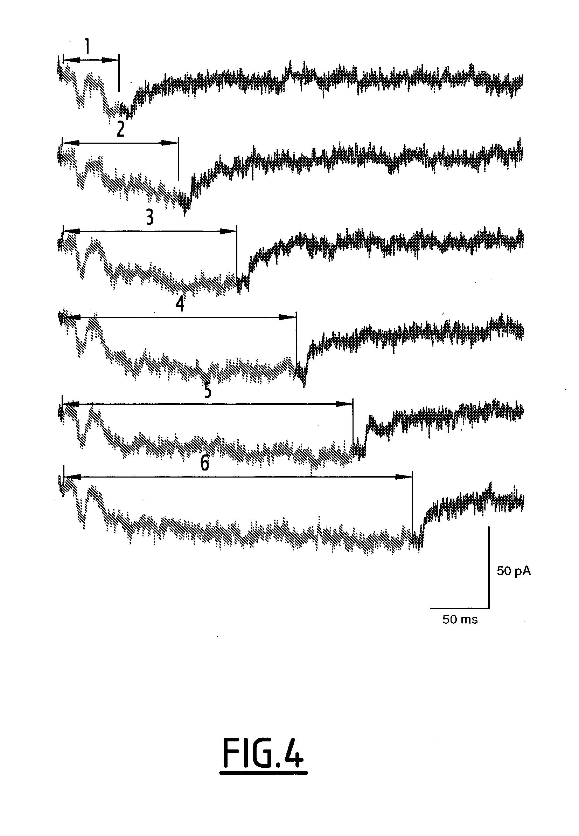

La figure 4 représente les courants enregistrés sur des cellules PC12 cultivées sur une rétine comprenant 5 couches de nanotubes de dioxyde de titane déposées par dépôt couche par couche et de l'or en réponse à des flashs lumineux de 50 à 300 ms en lumière bleue (lampe Xénon à 100 W, avec un filtre passe bande 450-490 nm) (exemple 2).

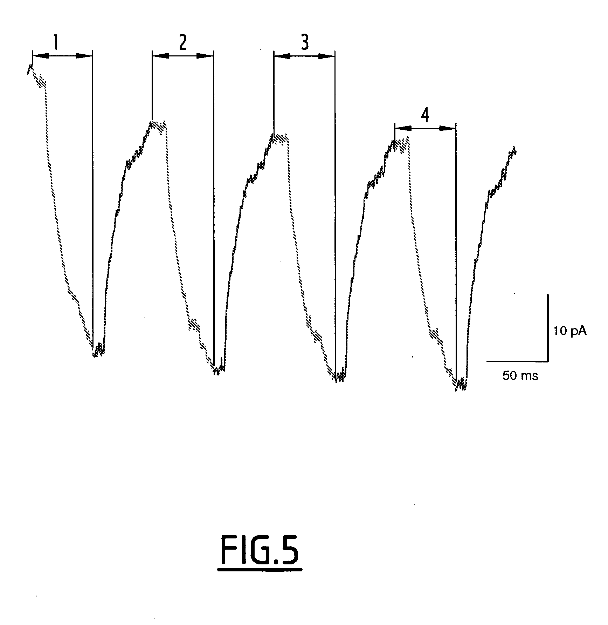

La figure 5 représente les courants enregistrés sur des cellules PC12 cultivées sur une rétine comprenant 2 couches de dioxyde de titane (P-25 Degussa-Evonik^{®}) déposées par dépôt couche par couche et de l'or en réponse à des flashs lumineux de 50 ms appliqués à une fréquence de 10 Hz en lumière verte (filtre passe bande 530-560 nm) (exemple 2).

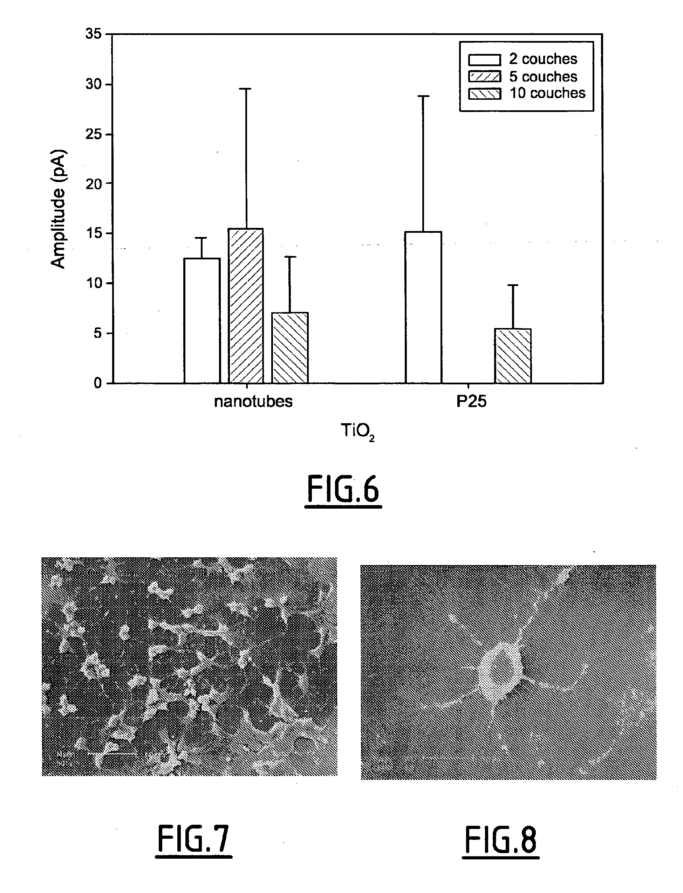

La figure 6 représente l'intensité des courants (en pA) mesurés sur des cellules PC12 cultivées sur une rétine comprenant 2, 5 ou 10 couches de dioxyde de titane, sous forme de nanotubes ou non, déposées par dépôt couche par couche et de l'or en réponse à des flashs lumineux en lumière bleue de 300 ms (450-490 nm, 320 mW/cm²) (exemple 2).
Les figures 7 et 8 représentent les images observées en microscopie électronique à balayage de cellules PC12 cultivées sur des rétines comprenant 5 couches de nanotubes de TiO₂ et de l'or (microscope Philips^{®} XL 20).

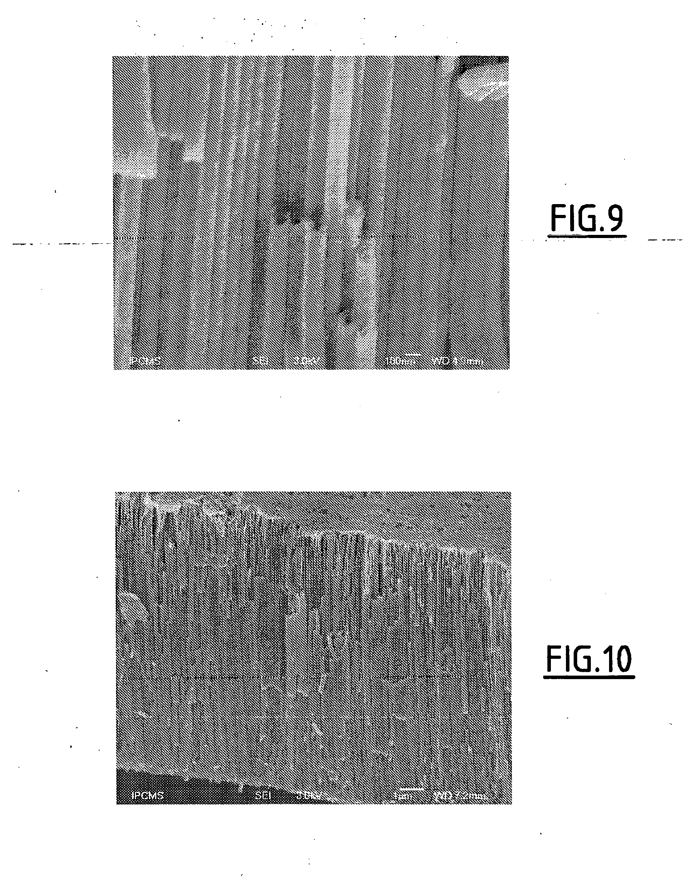

Les figures 9 et 10 représentent des images obtenues en microscopie (Microscope électronique à balayage JEOL-JSM-6700F) pour des parties en matériau photovoltaïque en TiO₂ sous forme de nanotubes alignés les uns par rapport aux autres sur un substrat en titane synthétisées avec les paramètres suivants : électrolyte à base d'éthylène glycol avec 2 % volumique d'eau et 0,3% massique de NH₄F. L'anodisation à été effectuée avec une tension de 45 V pendant 2 heures. Les images ont été réalisées après un traitement thermique des échantillons sous flux d'air à 450°C pendant 6h (exemple 4a).

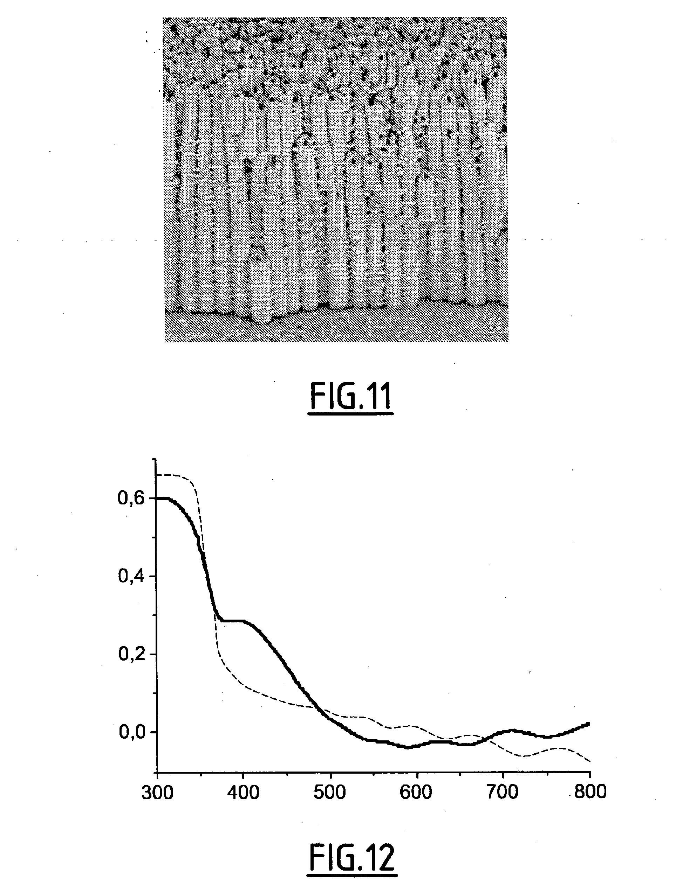

La figure 11 représente une image obtenue en microscopie (Microscope électronique à balayage JEOL-JSM-6700F) pour une partie en matériau photovoltaïque en TiO₂ sous forme de nanotubes alignés les uns par rapport aux autres sur un substrat en silicium (111) synthétisée en suivant le protocole de l'exemple 4b.
La figure 12 représente les spectres UV-visible (absorbance en u.a. en fonction de la longueur d'onde en nm) des matériaux photovoltaïques des exemples 4a) (trait en pointillé) et 5 (trait plein) (obtenus avec un spectromètre UV-visible CARY 100 SCAN de la marque Varian, équipé d'une sphère intégrante Labsphere DRA-CA-301).
La figure 13 représente une image obtenue en microscopie en éclairage rasant (Obj. 40x) du matériau de l'exemple 6 obtenu après usinage laser.
La figure 14 représente schématiquement le parcours que les électrons photogénérés suivent pour traverser une rétine comprenant des parties en matériau photovoltaïque sous forme de grains ou de nanotubes enchevêtrés (à gauche) ou sous forme de nanotubes alignés les uns par rapport aux autres (à droite).

### EXEMPLES

Les exemples 1 à 6 concernent la préparation et l'utilisation d'un pixel d'une rétine artificielle selon l'invention. En effet, les rétines artificielles exemplifiées ne comportent pas de partie en matériau isolant. Ces exemples démontrent qu'un semi-conducteur à base de dioxyde de titane peut être utilisé comme couche en matériau photovoltaïque dans une rétine artificielle.

### EXEMPLE 1 : Rétines comprenant du dioxyde de titane et de l'or

### Exemple 1a : à partir de nanotubes de TiO₂

Les nanotubes ont été synthétisés par traitement hydrothermal à 150°C pendant 72 heures de 700 mL d'une solution aqueuse d'hydroxyde de sodium à 10 mol/L. comprenant 7 g de TiO₂ (Degussa^{®} P-25). Le précipité blanc obtenu a alors été maintenu sous vide au dessiccateur à 500 °C pendant 18 heures. Les nanotubes obtenus ont alors été rendus déficients en oxygène par traitement sous flux d'hydrogène pendant 2 heures à 350 °C, cette température ayant été atteinte par une montée en température de 5 °C par minute. Ce traitement rendre les nanotubes activables par une partie du visible du spectre solaire.

Les nanotubes ont alors été dispersés dans une solution éthanol / eau distillée (50/50 en volume) ayant un pH ajusté à 9 sous agitation pendant une heure pour obtenir une solution de nanotubes dispersés.

Après prétraitement d'une tranche de silicium par nettoyage par une solution d'acide chlorhydrique à pH 3, puis par l'acétone et enfin par l'éthanol sous agitation orbitalaire (10 minutes à 100 tours par minute pour chaque nettoyage) et séchage de la tranche pendant 5 minutes sous flux d'air chaud, un dépôt couche par couche (« layer by layer » LbL en anglais) été réalisé comme suit :
1) Cette tranche a alors été immergée dans 50 mL d'une solution aqueuse de polyéthylèneimine à 8 g/L pendant 10 minutes puis rincée par immersion dans 50 mL d'eau distillée pendant 10 minutes.
2) La tranche a ensuite été immergée dans 50 mL d'une solution aqueuse de chlorure de diallyldiméthylammonium à 2% massique pendant 10 minutes puis rincée par immersion dans 50 mL d'eau distillée pendant 10 minutes.
3) Puis, la tranche a été immergée dans 50 mL de la solution de nanotubes dispersés pendant 10 minutes puis rincée par immersion dans 50 mL d'eau distillée pendant 10 minutes.

Les étapes 2) et 3) peuvent être répétées pour obtenir des couches multiples. Dans l'exemple, ces étapes ont été répétées pour obtenir 5 et 10 couches de nanotubes de TiO₂ sur la tranche de silicium.

Après dépôt couche par couche, les substrats sont séchés à l'air à température ambiante, puis un dépôt d'épaisseur nanométrique d'or est obtenu par dépôt physique en phase vapeur (« physical vapor déposition » PVD en anglais) durant 2 minutes avec une tension appliquée de + 22 mV à une pression de 10⁻⁶ bar.

### Exemple 1b : à partir de TiO₂ commercial

La dispersion de TiO₂ est obtenue par mise en suspension de 0,4 g de TiO₂ (P-25 Degussa-Evonik^{®}) dans 100 mL d'une solution éthanol / eau distillée (50/50 en volume) ayant un pH ajusté à 9 sous agitation pendant une heure.

Les étapes ultérieures de prétraitement d'une tranche de silicium, de dépôt couche par couche de TiO₂ puis de dépôt d'une couche d'épaisseur nanométrique d'or ont été effectuées en suivant le protocole de l'exemple 1 a), en remplaçant la solution de nanotubes dispersés par la dispersion de TiO₂ pour le dépôt couche par couche de TiO₂.

EXEMPLE 2 : Photoréponse de cellules PC12 cultivées sur une rétine de TiO₂ recouverte d'or.

Des enregistrements ont été réalisés en utilisant une technique électrophysiologique (dite de « patch clamp » en anglais) d'enregistrement des courants ioniques transitant à travers les membranes cellulaires de cellules de la lignée PC 12 (issue d'un phéochromocytome de rat. Ces cellules sont couramment utilisées comme modèle cellulaire en électrophysiologie car leur culture est facile et elles peuvent être différenciées en neurones par simple ajout d'un facteur de croissance des nerfs (le NGF) dans le milieu de culture) (Greene & Tischler, P.N.A.S 1976, 73(7) : 2424-8), cultivées sur des lamelles de verre recouvertes :
- de nanotubes de TiO₂ (lamelles obtenues après dépôt couche par couche de 2, 5 ou 10 couches de nanotubes de TiO₂, puis recouvertes d'or), ou
- de TiO₂ (P-25 Degussa-Evonik^{®}) (lamelles obtenues après dépôt couche par couche de 2, 5 ou 10 couches de TiO₂, puis recouvertes d'or),
dans un milieu de culture cellulaire classique (milieu de Dulbecco (DMEM (Dulbecco/Vogt modified Eagle's minimal essential medium)) et 10% de sérum de veau foetal). Pour augmenter l'adhérence des cellules, les lamelles ont été recouvertes de poly-lysine (un polymère de l'acide aminé L-lysine). Dans des conditions physiologiques de pH, la polylysine forme une couche chargée positivement sur la rétine artificielle, qui attire les têtes hydrophiles des lipides membranaires, chargées négativement. La configuration « cellule entière » a été mise en oeuvre, afin de mesurer en direct l'ensemble des courants ioniques passant à travers la membrane cellulaire. Le potentiel imposé était de -70 mV.

Les figures 4 et 5 représentent les courants ioniques enregistrés sur des cellules PC12 cultivées sur deux rétines TiO₂/or, plus précisément :
- comprenant 5 couches de nanotubes de dioxyde de titane déposées par dépôt couche par couche et recouvertes d'or en réponse à des flashs lumineux de 50 à 300 ms en lumière bleue,
- comprenant 2 couches de dioxyde de titane (P-25 Degussa-Evonik^{®}) déposés par dépôt couche par couche et recouvertes d'or en réponse à des flashs lumineux de 50 ms appliqués à une fréquence de 10 Hz en lumière verte.

Le courant transmembranaire est la somme des courants créés par le passage des ions à travers la membrane de la cellule. Plus précisément, il existe une différence de potentiel entre l'intérieur et l'extérieur de toute cellule vivante. Les ions des milieux intra et extracellulaires se déplacent de part et d'autre de la membrane, selon la résultante de ce gradient de potentiel et de leur gradient de concentration par l'intermédiaire des canaux ioniques. Dans une cellule en bonne santé, l'intérieur de la cellule est plus négatif que l'extérieur. Si on maintient la cellule a proximité de son potentiel d'équilibre (-70 mV dans l'exemple), il y a donc très peu de courant passant à travers la membrane. Le flux net des ions est alors nul car la répartition de chaque espèce ionique est proche de son équilibre électrochimique. Par contre, si le potentiel au contact de la cellule est modifié, dans l'exemple par passage d'un courant à travers le TiO₂, la cellule n'est plus à l'équilibre : le flux net des ions à travers la membrane n'est plus nul, et un courant est mesuré. Celui-ci est négatif, c'est-à-dire qu'il y a une entrée nette de charges positives dans la cellule.

A l'obscurité, le courant transmembranaire est identique à celui enregistré pour des cellules cultivées sur des lamelles de verre (en l'absence de dioxyde de titane), qui servent de contrôle.

Par contre, un courant entrant est mesuré en réponse à un flash lumineux pour les cellules cultivées sur les rétines TiO₂/or, ce qui n'est pas les cas pour les cellules cultivées sur des lamelles de verre. La durée de la réponse augmente avec celle du flash (figure 4). Des flashs de 50 ms appliqués à une fréquence de 10 Hz provoquent des courants comparables (figure 5), sans dégradation de l'état de la cellule.

La figure 6 représente l'intensité des courants (en pA) mesurés en réponse à des flashs lumineux en lumière bleue de 300 ms sur des cellules PC12 cultivées en fonction de la nature de la rétine : rétine comprenant 2, 5 ou 10 couches de dioxyde de titane, sous forme de nanotubes ou non, et une couche en matériau conducteur en or. Les intensités sont similaires que le TiO₂ soit déposé sous forme de nanotube ou non. Les intensités mesurées sont plus importantes pour 2 ou 5 couches de TiO₂ que pour 10 couches.

### EXEMPLE 3 : Biocompatibilité d'une rétine artificielle comprenant du TiO₂ et de l'or.

La biocompatibilité du dioxyde de titane (recouvert ou non d'or) a été testée en comparant la vitesse de croissance de cellules NG108-15 sur des rétines artificielles préparées selon l'exemple 1 a et 1 b ou sur des lamelles de verre (contrôle), dans les deux cas recouverts de poly-lysine afin de favoriser l'adhérence cellulaire, dans un milieu DMEM / 1 % de sérum de veau foetal afin de ralentir la croissance des cellules et d'accentuer leur phénotype neuronal. Que ce soit sur les lamelles de verre (contrôle) ou sur les différentes rétines comprenant du TiO₂, les cellules ensemencées à 10³ / cm² arrivent à confluence au bout de 12 jours. Les potentiels d'action enregistrés pour les cellules sur verre et sur les rétines comprenant du TiO₂ sont également très proches. On observe par ailleurs que les cellules PC12 en croissance sur le TiO₂ forment de nombreuses extensions (figures 7 et 8). Pour réaliser les images en microscopie en balayage des figures 7 et 8, les cellules ont été fixées dans un tampon de cacodylate de sodium contenant 2,5% (masse/volume) de glutaraldéhyde, puis post-fixées 1 h dans le même tampon auquel a été ajouté 1% d'acide osmique. Les cellules ont ensuite été déshydratées, séchées, métallisées puis observée avec un microscope Philips^{®} XL 20.

L'ensemble de ces données montre que les rétines artificielles comprenant du TiO₂ présentent une bonne biocompatibilité, les rendant ainsi intéressantes pour une implantation.

### EXEMPLE 4 : Rétine comprenant un matériau photovoltaïque en TiO₂ sous forme de nanotubes alignés les uns par rapport aux autres

### Exemple 4a) : Substrat en titane

Avant l'expérience, une feuille de titane métallique a été nettoyée par décapage chimique dans l'eau Régale (mélange HNO₃/HCl : 3/1 en volume). Puis elle a été nettoyée par ultrasonication dans des bains successifs d'acétone, d'éthanol et d'eau ultrapure (10 minutes dans chaque solvant).

La feuille ainsi obtenue a été mise en place dans la cellule d'électrochimie et la contre-électrode a également été placée. La distance entre les deux électrodes était comprise entre 1 et 5 cm. L'électrolyte utilisé était à base d'éthylène glycol auquel de l'eau et du NH₄F (fluorure d'ammonium) ont été ajoutés en différentes proportions : des teneurs en eau comprises entre 0 et 20 % volumique en eau et des teneurs en NH₄F entre 0,1 et 5 % massique ont été mises en oeuvre. Dans les conditions testées, les paramètres optimaux de synthèse sont des teneurs en eau entre 1 et 2% volumique et 0,2 et 0,5% massique en NH₄F. Dans les conditions testées, le potentiel à appliquer entre les deux électrodes est de préférence compris entre 20 et 100 V, en particulier entre 40 et 60 V, pour synthétiser efficacement les nanotubes. La durée de synthèse peut être comprise entre 10 minutes et 100 heures. Les courtes durées (entre 15 minutes et 2 heures) ont permis la formation de nanotubes bien alignés les uns par rapport aux autres.

Les films de nanotubes de TiO₂ supportés sur du titane ainsi obtenus ont été rincés à l'eau ultrapure pour enlever tout l'électrolyte restant.

Un traitement thermique a lors été effectué pendant une durée de 1 à 12 h sous flux d'air à une température comprise entre 400 et 600 °C. Une durée de 2h est suffisante pour ce traitement thermique et une température de 450 °C est optimale. Un exemple typique des morphologies obtenues est présenté en figures 9 et 10. Les nanotubes obtenus par la méthode décrite ci-dessus, présentent un diamètre extérieur de l'ordre de 100 nm. Le diamètre intérieur varie entre 20 nm à la base des tubes et 90 nm au sommet. La longueur est fortement dépendante de la durée de synthèse. Pour 2 heures de synthèse, une longueur de 15 µm est typiquement obtenue.

### Exemple 4b) : Substrat en silicium (111)

### - Dépôt de titane sur un substrat en silicium par pulvérisation par magnétron

Une cible de titane métallique de grande pureté (99,99%) a été bombardée par les ions issus d'un plasma d'argon (Puissance plasma 300 W, flux argon 20 sccm - intensité de courant de 600 mA - température de 50 °C). Les atomes de titane ont été arrachés de la cible et se sont déposés sur le substrat en silicium (111) placé en vis-à-vis de la cible. Une couche de titane de 900 nm d'épaisseur a été déposée sur le silicium.

### - anodisation électrochimique

L'anodisation électrochimique du substrat en silicium sur lequel du titane a été déposé a été réalisé dans un électrolyte à base de glycérol avec 2% volumique d'eau et 0,5% massique de NH₄F. Les conditions d'anodisation optimale étaient de 35 V pendant 4 heures à une température de 25 °C. Le film de nanotubes de TiO₂ alignés obtenus est présenté en figure 11.

### EXEMPLE 5 : Rétine comprenant un matériau photovoltaïque en TiO₂ dopé à l'azote sous forme de nanotubes alignés les uns par rapport aux autres

La préparation des nanotubes par anodisation électrochimique a été réalisée sur une feuille de titane métallique de 1 x 1 cm², dans 100 mL d'une solution d'électrolyte de concentration 1 M obtenue comme suit : 6,742 mL d'H₃PO₄ concentré (Fluka, 85%) a été complétée à 100 mL par de l'eau distillée, de manière à obtenir une concentration 1 M, avant que 1,167 mL d'HF (soit 0,5% massique) ne soit ajouté. L'électrolyte a été stabilisé à pH 5 avec 4,88 g de NaOH. 3,03 g d'urée N₂COH₄, soit une concentration d'urée dans le bain de 0,5 mol.L⁻¹, ont ensuite été ajoutés comme source d'azote. L'anodisation a été réalisée avec un potentiel de 20V pendant 4 heures.

Finalement un traitement thermique de 450 °C pendant 6 heures a été effectué sous flux d'azote (100 mL/min, rampe de montée de 2 °C/min).

Les spectres obtenus par spectroscopie UV-visible pour l'exemple 4a) et pour l'exemple 5 sont représentés à la figure 12. La présence d'azote dans les nanotubes permet d'avoir l'absorption d'une partie du rayonnement visible (longueur d'onde > 400 nm), qui se traduit par l'apparition d'un deuxième seuil d'absorption à 530 nm.

### Exemple 6 : Microgravure d'un matériau photovoltaïque permettant la pixellisation de la rétine artificielle

Le procédé de préparation de la rétine artificielle selon l'invention comprend une étape (étape c) consistant à insérer au moins une partie en matériau isolant dans la couche en matériau photovoltaïque et dans la couche en matériau conducteur). Cette étape comprend une étape de microgravure, de manière à obtenir en inverse le marquage désiré.

Dans cet exemple, la microgravure a été réalisée par ablation laser du matériau de l'exemple 4a) à l'air libre sans gaz d'assistance, selon les paramètres suivants :

| | |
|---|---|
| Optique de focalisation | LMU-15X-266 OFR |
| Laser | Nd :YAG QS 266nm/10ns HIPPO |
| Energie par impulsion | < 10 muW |
| Taux de répétition | 50 kHz |
| Temps d'exposition minimal | 1s |

Des traits de 200 µm de longueur et de 15 µm de largeurs ont ainsi été tracés à des vitesses de l'ordre de 100µm/s, comme illustré en figure 13. Répéter l'opération en opérant une rotation orthogonale permet de dessiner des rectangles ou carrés, chaque rectangle/carré obtenu étant un des pixels de la rétine artificielle.

## Revendications

1. Rétine artificielle comprenant :
(i) un substrat,
(ii) une première couche disposée sur ledit substrat comprenant des parties en matériau photovoltaïque séparées par au moins une partie en matériau isolant,
(iii) une deuxième couche disposée sur ladite première couche et comprenant des parties en matériau conducteur séparées par au moins une partie en matériau isolant, **caractérisée en ce que** le matériau photovoltaïque comprend un semiconducteur à base de dioxyde de titane sous forme de nanotubes.

2. Rétine artificielle selon la revendication 1, dans laquelle le dioxyde de titane est sous forme de rutile, d'anatase, de brookite, de srilankite, de TiO₂ α ou de TiO₂ β.

3. Rétine artificielle selon la revendication 1 ou 2, dans laquelle les nanotubes sont alignés les uns par rapport aux autres.

4. Rétine artificielle selon l'une quelconque des revendications 1 à 3, dans laquelle les nanotubes sont modifiés avec un sensibilisateur.

5. Rétine artificielle selon l'une quelconque des revendications 1 à 4, dans laquelle les nanotubes sont dopés avec de l'azote.

6. Rétine artificielle selon l'une quelconque des revendications 1 à 5, dans laquelle le matériau conducteur est un diamant conducteur, du nitrure de titane, de l'oxyde d'iridium, un polymère conducteur ou un métal, tel que l'or ou le platine.

7. Rétine artificielle selon l'une quelconque des revendications 1 à 6, dans laquelle le matériau isolant de la première et/ou de la deuxième couche est un diamant isolant, une céramique ou un polymère isolant.

8. Rétine artificielle selon l'une quelconque des revendications 1 à 7, dans laquelle les parties en matériau photovoltaïque et les parties en matériau conducteur sont superposées les unes aux autres.

9. Rétine artificielle selon l'une quelconque des revendications 1 à 8, dans laquelle les surfaces des parties en matériau conducteur sont inférieures aux surfaces des parties en matériau photovoltaïque.

10. Rétine artificielle selon l'une quelconque des revendications 1 à 9, dans laquelle l'épaisseur des parties en matériau conducteur est supérieure, notamment de 3 à 50 µm, à l'épaisseur de la partie isolante de la deuxième couche.

11. Procédé de préparation d'une rétine artificielle selon l'une quelconque des revendications 1 à 10, comprenant les étapes consistant à :
a) déposer un semiconducteur à base de dioxyde de titane sous forme de nanotubes sur un substrat pour former une couche en matériau photovoltaïque,
b) déposer une couche en matériau conducteur sur la couche en matériau photovoltaïque obtenue à l'étape a) pour former une couche en matériau conducteur,
c) insérer au moins une partie en matériau isolant dans la couche en matériau conducteur et dans la couche en matériau photovoltaïque pour former la première et la deuxième couche.

12. Procédé selon la revendication 11, dans lequel l'étape a) est réalisé par dépôt couche par couche.

13. Procédé selon l'une quelconque des revendications 11 à 12, dans lequel l'étape b) est réalisée par dépôt physique ou chimique en phase vapeur ou par lithographie.

14. Procédé selon l'une quelconque des revendications 11 à 13, dans lequel l'étape c) comprend une étape de microgravure ou de lithographie.

## Patentansprüche

1. Künstliche Netzhaut umfassend:
(i) ein Substrat,
(ii) eine erste auf dem Substrat abgeschiedene Schicht, die Bestandteile aus einem photovoltaischen Material umfasst, wobei die Bestandteile durch mindestens ein Bestandteil aus isolierendem Material getrennt sind,
(iii) eine auf der ersten Schicht abgeschiedene zweite Schicht, die Bestandteile aus einem leitenden Material enthält, wobei die Bestandteile durch mindestens einen Bestandteil aus isolierendem Material getrennt sind,
**dadurch gekennzeichnet,**
**dass** das photovoltaische Material einen Halbleiter auf Basis von Titandioxid in Form von Nanoröhrchen umfasst.

2. Künstliche Netzhaut nach Anspruch 1, bei der das Titandioxid in Form von Rutil, Anatas, Brokit, Srilankit, TiO₂ α oder TiO₂ β vorliegt.

3. Künstliche Netzhaut nach Anspruch 1 oder 2, bei der die Nanoröhrchen zueinander ausgerichtet sind.

4. Künstliche Netzhaut nach einem beliebigen der Ansprüche 1 bis 3, bei der die Nanoröhrchen mit einem Sensibilisator modifiziert sind.

5. Künstliche Netzhaut nach einem beliebigen der Ansprüche 1 bis 4, bei der die Nanoröhrchen mit Stickstoff dotiert sind.

6. Künstliche Netzhaut nach einem beliebigen der Ansprüche 1 bis 5, bei der das leitende Material ein leitender Diamant, Titannitrid, Iridiumoxid, ein leitendes Polymer oder ein Metall, wie Gold oder Platin, ist.

7. Künstliche Netzhaut nach einem beliebigen der Ansprüche 1 bis 6, bei der das isolierende Material der ersten und/oder der zweiten Schicht ein isolierender Diamant, eine Keramik oder ein isolierendes Polymer ist.

8. Künstliche Netzhaut nach einem beliebigen der Ansprüche 1 bis 7, bei der die Bestandteile aus einem photovoltaischen Material und die Bestandteile aus leitendem Material übereinander angeordnet sind.

9. Künstliche Netzhaut nach einem beliebigen der Ansprüche 1 bis 8, bei der die Flächen der Bestandteile aus leitendem Material geringer als die Flächen der Bestandteile aus einem photovoltaischen Material sind.

10. Künstliche Netzhaut nach einem beliebigen der Ansprüche 1 bis 9, bei der die Dicke der Bestandteile aus einem leitenden Material größer ist, insbesondere um 3 bis 50 µm, als die Dicke des isolierenden Bestandteils der zweiten Schicht.

11. Verfahren zum Herstellen einer künstlichen Netzhaut nach einem beliebigen der Ansprüche 1 bis 10, die folgenden Schritte umfassen:
a) Abscheiden eines Halbleiters auf der Basis von Titandioxid in Form von Nanoröhrchen auf einem Substrat, um eine Schicht aus einem photovoltaischen Material zu bilden,
b) Abscheiden einer Schicht aus einem leitenden Material auf der Schicht aus einem photovoltaischen Material, die im Schritt a) erhalten wurde, um eine Schicht aus leitendem Material zu bilden,
c) Einfügen mindestens eines Bestandteils aus einem isolierenden Material in die Schicht aus leitendem Material und in die Schicht aus einem photovoltaischen Material, um die erste und die zweite Schicht zu bilden.

12. Verfahren nach Anspruch 11, bei dem der Schritt a) durch eine schichtweise Abscheidung realisiert wird.

13. Verfahren nach einem beliebigen der Ansprüche 11 bis 12, bei dem der Schritt b) durch eine physikalische oder chemische Dampfabscheidung oder durch Lithographie realisiert ist.

14. Verfahren nach einem beliebigen der Ansprüche 11 bis 13, bei dem der Schritt c) einen Schritt der Mikrogravur oder der Lithographie umfasst.

## Claims

1. An artificial retina comprising:
(i) a substrate;
(ii) a first layer placed onto the said substrate comprising photovoltaic material portions separated by at least one insulating material portion;
(iii) a second layer placed onto the said first layer and comprising conductive material portions separated by at least one insulating material portion,
**characterized in that** the photovoltaic material comprises a titanium dioxide semiconductor in the form of nanotubes.

2. The artificial retina according to claim 1 wherein the titanium dioxide is in the form of rutile, anatase, brookite, srilankite, TiO₂ α or TiO₂ β.

3. The artificial retina according to claim 1 or 2, wherein the nanotubes are aligned relative to one another.

4. The artificial retina according to any one of claims 1 to 3 wherein the nanotubes are modified with a sensitizer.

5. The artificial retina according to any one of claims 1 to 4 wherein the nanotubes are doped with nitrogen.

6. The artificial retina according to any one of claims 1 to 5 wherein the conductive material is a conductive diamond, titanium nitride, iridium oxide, a conductive polymer or a metal such as gold or platinum.

7. The artificial retina according to any one of claims 1 to 6 wherein the insulating material of the first and/or second layer is an insulating diamond, a ceramic or an insulating polymer.

8. The artificial retina according to any one of claims 1 to 7 wherein the portions in photovoltaic material and the portions in conductive material are superimposed over each other.

9. The artificial retina according to any one of claims 1 to 8 wherein the surfaces of the portions in conductive material are smaller than the surfaces of the portions in photovoltaic material.

10. The artificial retina according to any one of claims 1 to 9 wherein the thickness of the portions in conductive material is greater, in particular by 3 to 50 µm, than the thickness of the insulating portion of the second layer.

11. A method for preparing an artificial retina according to any one of claims 1 to 10, comprising the steps of:
a) depositing a titanium dioxide semiconductor in the form of nanotubes on a substrate to form a layer in photovoltaic material;
b) depositing a layer in conductive material on the layer in photovoltaic material obtained at step a) to form a layer in conductive material;
c) inserting at least one portion of insulating material in the layer of conductive material and in the layer of photovoltaic material to form the first and second layer.

12. The method according to claim 11 wherein step a) is performed using layer-bylayer deposit.

13. The method according to claim 11 or 12 wherein step b) is conducted by physical vapor deposition or chemical vapor deposition or by lithography.

14. The method according to any one of claims 11 to 13 wherein step c) comprises a micro-etching or lithography step.
